# FASCICULE DE BREVET EUROPEEN

(11) **EP 0 327 776 B1**
(45) Date de publication et mention de la délivrance du brevet: **01.06.1994**
(21) Numéro de dépôt: 88420036.1
(22) Date de dépôt: 08.02.1988
(51) Int. Cl.: A61K 7/16

(54) **Pâte dentifrice à base d'argile**
Zahnpasta mit Tonerde
Clay-based toothpaste

(43) Date de publication de la demande: 16.08.1989
(73) Titulaire: Rigaud, Michel, F-69003 Lyon (FR)
(72) Inventeur: Rigaud, Michel, F-69003 Lyon (FR)

(56) Documents cités:
- FR-A- 1 022 172
- FR-B- 1 594 709

## Description

La présente invention est relative à une pâte dentifrice d'un type nouveau possédant à la fois des qualités d'hygiène et des propriétés médicamenteuse.

On sait que le problème des pâtes dentifrices consiste à réaliser un mélange capable de nettoyer les dents lorsqu'on les brosse, alors que par ailleurs ce mélange doit constituer une pâte qui conserve dans le temps la plasticité nécessaire.

On connaît par ailleurs les propriétés de l'argile, qui est couramment utilisée en cosmétologie ou pour des produits médicaux, d'hygiène ou de beauté. Par exemple, il est connu d'utiliser l'argile en application sur la peau, ou comme dentifrice.

Les dentifrices à l'argile peuvent se présenter ou bien sous la forme d'une poudre, ou bien sous la forme d'une pâte à l'eau. En pratique, on constate qu'une telle pâte à l'eau ne se conserve pas, car, à l'air libre, elle sèche rapidement et durcit par évaporation de l'eau.

D'autre part on connaît des pâtes dentifrices renfermant de l'argile et de la glycérine avec d'autres constituants, comme par exemple les compositions décrites dans FR - A -1 022 172 et FR - A - 1 594 709.

La présente invention est fondée sur cette constatation que le mélange d'argile et de glycérine, à l'exclusion de tout autre principe actif, peut constituer une pâte dentifrice présentant une action efficace, à la fois curative et préventive, sur la plaque dentaire.

Cette propriété du mélange en question peut s'expliquer par une interaction synergique entre l'argile et la glycérine, dont le mélange présente des propriété distinctes de celles de ses constituants pris isolément.

En effet :
- l'argile seul serait capable de nettoyer les dents, mais en les rayant et en utilisant un effet électrostatique connu ;
- la glycérine seule produit un effet cicatrisant et évite que les dents ne soient rayées par l'argile.

La pâte dentifrice selon l'invention se caractérise essentiellement en ce qu'elle est formée par un mélange homogène d'argile, de glycérine et d'eau dans les proportions suivantes:
- pour 100 g d'argile
- de 50 à 150 cm³ de glycérine
- de 10 à 50 cm³ d'eau.

Suivant un mode de réalisation préféré, ces proportions sont les suivantes :
- environ 100 g d'argile, en poudre ou en bloc ;
- environ 50 cm³ de glycérine
- environ 20 cm³ d'eau.

La pâte dentifrice selon l'invention peut également renfermer du sel marin préalablement dissout dans l' eau mise en oeuvre.

Bien entendu, il est possible de rajouter à ce mélange divers adjuvants ou colorants présents en faible quantité, et notamment :
- un colorant ;
- des essences ou extraits divers pour modifier le goût du dentifrice.

Le mélange ainsi constitué permet de conserver la pâte d'argile à l'état plastique, état qui sera plus ou moins plastique ou pâteux selon les quantités de glycérine ou d'eau en présence.

Pour la réalisation de la pâte dentifrice selon l'invention, la glycérine peut être mélangée soit à de l'argile sèche, soit à de l'argile déjà humide, l'argile pouvant se présenter en bloc ou en poudre.

Enfin, au mélange d'argile et de glycérine, on peut aussi incorporer de l'eau salée ou saumure. Par exemple, on peut prévoir, pour le mélange, les proportions suivantes :
- environ 100 g de poudre d'argile ;
- environ 50 cm³ de glycérine ;
- de 10 à 50 cm³ d'eau salée.

## Revendications

1. Pâte dentifrice à base d'argile, caractérisée en ce qu'elle est constituée d'un mélange homogène d'argile, de glycérine et d'eau, renfermant pour 100 g d'argile, 50 à 150cm³ de glycérine et 10 à 50 cm³ d'eau.

2. Pâte dentifrice selon la revendication 1, caractérisée en ce qu'elle renferme en outre du sel marin.

3. Pâte dentifrice selon la revendication 1 ou la revendication 2, caractérisée en ce qu'elle contient en outre des produits pour en modifier le goût et la couleur choisis dans le groupe des colorants, essences et extraits.

## Claims

1. Clay based toothpaste characterised in that it is composed of a homogeneous mixture of clay, glycerin and water, containing for 100g clay, 50 to 150 cm³ of glycerin and 10 to 50 cm³ of water.

2. Toothpaste according to claim 1, characterised in that it contains, furthermore, sea salt.

3. Toothpaste according to claim 1 or claim 2, characterised in that it contains, furthermore, products for altering the taste and colour chosen from the group of colourings, essences and extracts.

## Patentansprüche

1. Zahnpasta mit Tonerde, **dadurch gekennzeichnet,** daß sie aus einer homogenen Mischung aus Tonerde, Glycerin und Wasser besteht, wobei auf 100 g Tonerde 50 bis 150 cm³ Glycerin und 10 bis 50 cm³ Wasser kommen.

2. Zahnpasta nach Anspruch 1, **dadurch gekennzeichnet,** daß sie außerdem Meersalz enthält.

3. Zahnpasta nach einem der Ansprüche 1 oder 2, **dadurch gekennzeichnet,** daß sie außerdem Substanzen zur Beeinflussung des Geschmacks und der Farbe enthält, welche aus der Gruppe der Farbstoffe, Essenzen und Extrakte ausgewählt sind.
